# FASCICULE DE BREVET EUROPEEN

(11) **EP 1 730 505 B1**
(45) Date de publication et mention de la délivrance du brevet: **29.06.2016**
(21) Numéro de dépôt: 05736918.3
(22) Date de dépôt: 04.03.2005
(51) Int. Cl.: C12M 1/34, G01N 15/06, G01N 33/487, G01N 21/53

(54) **PROCEDE ET DISPOSITIF POUR MESURER ET CARACTERISER EN LIGNE UNE BIOMASSE DANS UNE FERMENTATION AVEC DES BACTERIES LACTIQUES**
VERFAHREN UND VORRICHTUNG ZUR VERMESSUNG UND CHARAKTERISIERUNG EINER BIOMASSE IN EINEM MILCHSÄUREBAKTERIEN-FERMENTATIONSVERFAHREN
METHOD AND DEVICE FOR THE MEASUREMENT AND ON-LINE CHARACTERISATION OF A BIOMASS IN A LACTIC ACID BACTERIA FERMENTATION PROCESS

(30) Priorité: 05.03.2004 FR 0402368
(43) Date de publication de la demande: 13.12.2006
(73) Titulaire: Université Montpellier II, 34095 Montpellier Cedex 05 (FR); Compagnie Gervais Danone, 75009 Paris (FR)
(72) Inventeur: ARNOUX, Anne-Solenn, F-34090 Montpellier (FR); PREZIOSI-BELLOY, Laurence, F-34000 Montpellier (FR); ESTEBAN, Geoffrey, F-30000 Nimes (FR); TEISSIER, Philippe, F-91120 Palaiseau (FR); BARBEAU, Jean-Yves, F-91430 Igny (FR); GHOMMIDH, Charles, F-34980 Saint Gely du Fesc (FR)
(74) Mandataire: Pontet Allano & Associes
(86) Numéro de dépôt international: PCT/FR2005/000531
(87) Numéro de publication internationale: WO 2005/085818

(56) Documents cités:
- WO-A-01/79828
- WO-A-03/069334
- US-A- 4 810 650
- US-A- 4 893 935
- US-A- 5 243 409

## Description

La présente invention concerne un procédé pour mesurer et caractériser en ligne une biomasse dans un processus de fermentation de bactéries lactiques. Elle vise également un dispositif mettant en oeuvre ce procédé, ainsi qu'un procédé de pilotage d'un système de fermentation, mettant en oeuvre un tel dispositif.

On connaît déjà des procédés de détermination de la concentration de biomasse dans un réacteur de fermentation, qui mettent en oeuvre des mesures de capacitance ou des mesures optiques.

Cependant, alors que la biomasse est, d'évidence, la variable dont la connaissance est la plus importante pour la maîtrise des processus fermentaires, les capteurs pour la mesurer sont aujourd'hui relativement rares, chers, et souvent méconnus. Une des raisons principales est probablement le caractère relativement flou de l'idée de biomasse: selon les domaines d'application, les opérateurs mesurent le volume cellulaire, la masse sèche, le nombre de cellules, le nombre de colonies après culture, la turbidité, voire même l'activité métabolique (respiration, vitesse d'acidification...). La correspondance des mesures obtenues par ces diverses méthodes est loin d'être constante, et évolue en fonction des conditions de culture. Toutes ne sont pas toujours utilisables, comme par exemple la mesure de la concentration de matière sèche après filtration lorsque le milieu est visqueux ou contient des particules en suspension.

La notion d'état physiologique est également délicate à définir : suivant les applications, elle fait référence à la position des cellules dans leur cycle végétatif, à leur état de viabilité, à leur morphologie... Les opérateurs décrivent pourtant leur culture comme étant en "bon (ou mauvais) état physiologique", en fonction de critères, pas toujours objectifs, généralement liés à l'évolution temporelle de la fermentation. On retrouve là un problème sémantique similaire à celui évoqué pour la biomasse. Parmi les moyens conventionnels d'évaluation de l'état physiologique d'une population, on peut citer:
la mesure de la morphologie cellulaire par microscopie (avec éventuellement l'appui de techniques d'analyse d'image). On peut mesurer la longueur (ou le diamètre) des cellules, l'état d'agrégation (cellules isolées, par paires...), l'état de division (ratio cellules en division/cellules isolées) ;
l'aptitude à la division, après culture sur milieu approprié (milieu gélosé, membrane...). On dénombre alors le nombre de colonies obtenues, et on calcule le ratio au nombre initial de cellules ;
le suivi par microscopie ou cytométrie de flux de la pénétration de marqueurs colorés, souvent fluorescents, et leur métabolisation éventuelle, qui permet d'évaluer la perméabilité membranaire et la présence d'activités physiologiques particulières ;
La mesure d'une activité métabolique globale (vitesse d'acidification, vitesse de consommation d'oxygène, de production de CO₂, divisée par la concentration de biomasse totale (généralement exprimée en concentration de matière sèche).

A coté des mesures conventionnelles, qui nécessitent généralement un échantillonnage, la mise en oeuvre de systèmes de mesure *in situ* commence à se populariser. Il existe essentiellement deux technologies concurrentes, basées soit sur les propriétés optiques des suspensions microbiennes, soit sur les propriétés diélectriques des cellules vivantes.

Les mesures optiques sont généralement basées sur l'évaluation simultanée de l'ensemble des phénomènes (absorption, réflexion, réfraction, diffraction) qui affectant les suspensions de microorganismes. Leur principal avantage est leur sensibilité aux faibles concentration de biomasse. Leur principal inconvénient est la détection de particules autres que la biomasse, et dans une moindre mesure, des cellules mortes. Ainsi, la document US 4 893 935 décrit un dispositif et un procédé pour déterminer une biomasse d'un milieu, à partir d'une mesure de densité optique du milieu.

Par ailleurs, les structures membranaires, qui empêchent la libre circulation des ions, confèrent aux cellules vivantes le comportement de petits condensateurs. Les mesures de capacitance (à distinguer des mesures d'impédance) permettent, en principe, une évaluation directe du volume cellulaire. Le signal obtenu dépend de la taille des cellules, celles de petite taille se polarisant plus rapidement que les cellules de grande taille. Le signal dépend également de l'état des membranes, les cellules en "mauvais état" ne se polarisant que faiblement, ce qui constitue le principal avantage des mesures de capacitance. On peut notamment citer l'article intitulé "On-line, real-time measurement of cellular biomass using dielectric spectroscopy » de Yardley et. al., publié dans "Biotechnology and Genetic Engineering Reviews", (2000) Vol.17. En poutre, les documents US 4 810 650 et WO 03/069334 décrivent un dispositif et un procédé pour déterminer une biomasse d'un milieu, à partir d'une mesure capacitive du milieu pour une pluralité de fréquences.

On constate donc que le problème posé est à la fois mal défini au plan sémantique, complexe et délicat à étudier en raison du manque de moyens d'investigation adaptés.

L'objectif de la présente invention est d'apporter une solution technique à ce problème en proposant un procédé de mesure de concentration en ligne d'une biomasse de bactéries lactiques en fermentation, qui fournisse simultanément *in situ* et en temps réel une évaluation de l'état physiologique des populations microbiennes contenues dans cette biomasse.

Cet objectif est atteint avec un procédé de mesure et de caractérisation en ligne d'une biomasse dans un processus de fermentation de bactéries lactiques en suspension dans un milieu selon la revendication 1.

De manière schématique, on peut écrire que la capacitance C et la densité optique D d'une suspension cellulaire sont des fonctions de la concentration de biomasse X, de la taille des cellules Φ et de leur état physiologique Ψ.

C = f (X, Φ, Ψ)

D = g (X, Φ)

En théorie, il devrait être possible de résoudre ce système, en réalisant les mesures de capacitance et de densité optique à différentes fréquences, de manière à disposer d'un nombre suffisant d'équations.

Une analyse temporelle des signaux peut également apporter une information sur l'état d'agrégation des cellules.

Les difficultés existent à différents niveaux : les fonctions f et g ne sont que partiellement définies et les variables Φ et Ψ sont en fait des distributions.

En couplant les mesures de spectroscopie d'impédance, de spectroscopie optique et de diffraction, et en s'appuyant sur les techniques modernes d'analyse de données (chimiométrie), il devrait être possible de remonter aux trois variables d'intérêt. Techniquement, il s'agit de mettre en correspondance d'une part toute l'information disponible, obtenue par les moyens conventionnels, et d'autre part, tous les signaux mesurables in situ, et ce, pour le plus grand nombre possible d'échantillons, correspondant aux situations les plus variées. Les techniques avancées d'analyse de données permettent ensuite de repérer les variables les plus porteuses d'information, et d'établir les corrélations les plus pertinentes.

On constate en pratique qu'il est relativement simple de croiser l'information apportée par plusieurs capteurs de principes différents pour obtenir une information utile à l'opérateur. On peut en effet limiter la dimension du problème en introduisant des hypothèses simplificatrices. Par exemple, si on postule que les cellules ont toujours la même taille (ou distribution de tailles) et sont toujours dans le même état physiologique, alors la mesure de capacitance C = f (X, Φ, Ψ) fournira directement une mesure de la concentration de biomasse. Cependant, si la taille des cellules varie en cours de fermentation, il n'est plus possible de savoir si la variation observée du signal est causée par une variation de la concentration cellulaire, ou une variation de la taille moyenne de la population ou une variation de l'état membranaire, ou toute combinaison des trois facteurs.

Ainsi, avec le procédé selon l'invention, il devient possible de mesurer la concentration de biomasse et d'évaluer simultanément l'état physiologique de populations microbiennes, en temps réel et *in situ.*

D'autres avantages et caractéristiques de l'invention apparaîtront à l'examen de la description détaillée d'un mode de mise en oeuvre nullement limitatif, et des dessins annexés sur lesquels :
- la figure 1 illustre l'évolution de la permittivité et de la densité optique au cours de deux cultures de *Lactobacillus casei*, et l'influence d'un changement de pH ;
- la figure 2 illustre une relation entre le rapport permittivité diélectrique/Densité optique et la longueur moyenne de *Lactobacillus casei* ; *et*
- la figure 3 illustre un exemple pratique de réalisation d'un dispositif de mesure de biomasse selon l'invention.

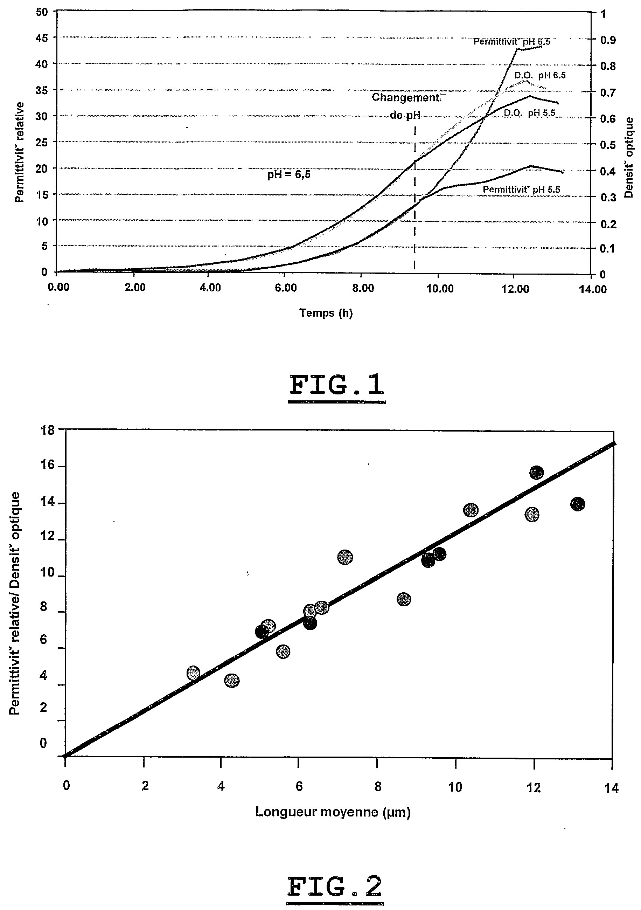

On va maintenant décrire, en référence aux figures précitées, un exemple de mise en oeuvre du procédé selon l'invention. Pour illustration, la figure 1 représente l'évolution temporelle de la permittivité diélectrique de deux suspensions bactériennes (*Lactobacillus casei*). Au cours d'une de ces cultures, le pH du milieu (régulé par addition contrôlée de potasse) a été abaissé. On constate une rupture de la courbe de permittivité correspondante, qui pourrait indiquer un arrêt de la croissance. Cependant, la mesure simultanée de la densité optique montre que la variation de pH ne produit qu'un léger ralentissement de la croissance. Des mesures indépendantes (hors-ligne, non présentées) de concentration de matière sèche confirment le fait que la variation du pH n'a affecté la croissance que de manière marginale. En fait, la variation du pH provoque une diminution progressive de la taille des bactéries, qui passe de 9.5 ± 0.5 µm avant l'abaissement du pH, à 5.5 ± 0.5 µm en fin de culture, avec une diminution correspondante du signal capacitif. Si on reporte le rapport des mesures Permittivité/Densité optique en fonction de la longueur moyenne des bactéries, mesurée en microscopie optique, on obtient une corrélation très satisfaisante, qui peut ensuite être utilisée en sens inverse pour évaluer la longueur moyenne à partir des mesures simultanées de densité optique et de permittivité diélectrique.

La figure 2 présente ainsi un ensemble de mesures réalisées au cours de six fermentations, au cours desquelles, la longueur moyenne des bactéries a été modifiée, en jouant soit sur le pH, soit sur la température de culture. Les points caractéristiques de forme triangulaire correspondent à des variations de longueur induites par des changements de température (35, 37 et 39°C).

Les variables de taille et d'état physiologique sont généralement liées au cours d'un même processus fermentaire, et on peut souvent se contenter d'une estimation de la taille moyenne, sans se préoccuper de la distribution de taille. Ceci ne signifie pas que ce type d'information (état et distribution) ne serait pas intéressant pour l'opérateur, mais s'appuie sur un constat simple : à ce jour, la plupart des fermentations sont conduites sans mesure en ligne de la biomasse, faute de moyens techniques adaptés (disponibilité, coût...)

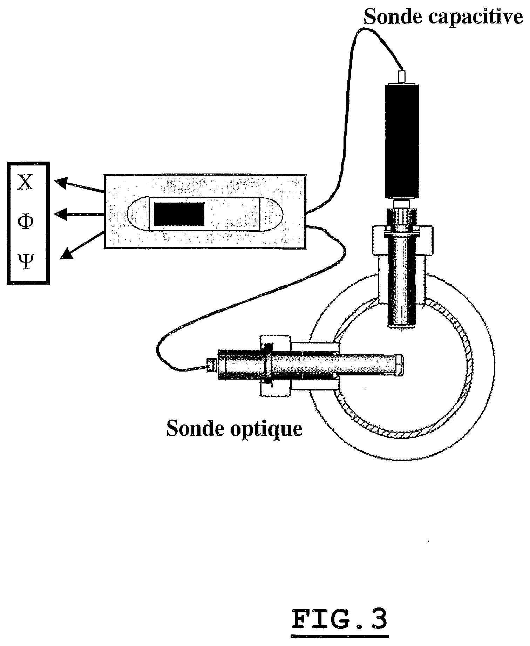

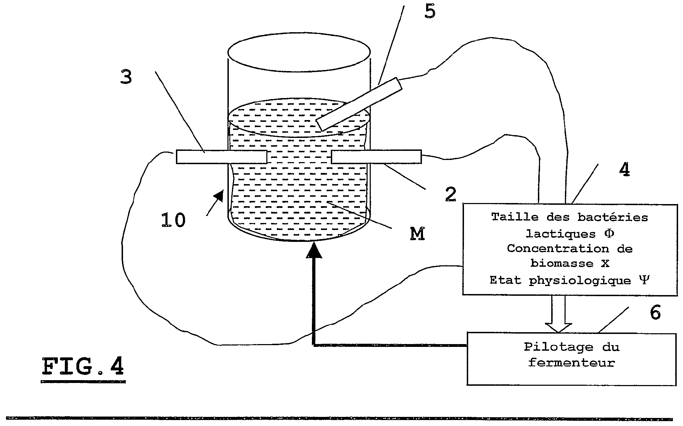

Ainsi, on a pu mesurer simultanément la concentration et la taille de bactéries lactiques en utilisant simultanément un analyseur de biomasse développé et commercialisé par la société Fogale Nanotech et une sonde optique Wedgewood (système mesurant dans le proche infra-rouge la densité optique de suspensions microbiennes), comme l'illustre la figure 3.

L'analyseur de biomasse comprend, comme l'illustre notamment le document WO 0179828, une sonde capacitive pour la détermination de la biomasse de cellules vivantes dont la surface cellulaire est inférieure ou égale à 10µm². Cette sonde capacitive est conçue pour être immergée dans un réacteur de fermentation contenant un milieu de cellules en suspension. Elle comprend une première paire d'électrodes d'intensité pour injecter un courant électrique dans le milieu, une seconde paire d'électrodes de tension pour relever la tension appliquée à ce milieu, et un circuit de mesure du courant électrique injecté. L'analyseur de biomasse comprend un conditionneur incluant un circuit pour fournir une tension alternative isolée galvaniquement à appliquer entre les électrodes d'intensité, et un circuit pour traiter des signaux représentatifs du courant injecté dans le milieu et de la tension relevée aux bornes des électrodes de tension, de façon à délivrer des signaux de mesure de la capacitance et de la conductance du milieu.

Le circuit de traitement comprend (i) un pont de mesure par méthode de zéro agencé pour traiter un signal image du courant injecté et un signal image de la tension relevée, ces deux signaux étant appliqués respectivement à une branche de référence et à deux branches d'opposition, et (ii) un circuit de commande automatique de ce pont à partir du signal de mesure de conductance.

Le pont de mesure par méthode de zéro est disposé en aval de circuits délivrant des signaux images respectivement du courant injecté et de la tension aux bornes de l'impédance à mesurer.

Le procédé de mesure en ligne de la concentration de biomasse selon l'invention est principalement dédié aux bactéries lactiques, notamment les lactobacilles, les streptocoques, les bifidobactéries.

Parmi les lactobacilles, on peut citer *Lactobacillus bulgaricus, Lactobacillus delbrueckii, lactobacillus casei.* Parmi les streptocoques, on peut citer *Streptococcus thermophilus.*

Parmi les bifidobacteries, on peut citer *Bifidobacterium animalis, Bifidobacterium longum, Bifidobacterium Breve, Bifidobacterium lactis.*

Les bactéries lactiques concernées par le procédé de mesure en ligne selon l'invention sont des cellules dont la surface n'excède pas 10 µm².

Le procédé de mesure en ligne selon l'invention peut être mis en oeuvre dans un bioréacteur ou fermenteur, de type industriel ou de laboratoire. Il peut être appliqué à des fermentations classiques en batch ou en milieu continu. Il peut aussi être mis en oeuvre pour le suivi de fermentations de type « fedbatch », notamment dans un procédé de pilotage d'un système de fermentation de bactéries lactiques régulé par ajustement de paramètres d'action pour obtenir une biomasse satisfaisant à au moins un critère cible déterminé en termes de taille de cellules ou d'activité physiologique. Au moins un de ces paramètres d'action peut être calculé à partir d'au moins un paramètre d'état mesuré au moyen d'un dispositif de mesure en ligne selon l'invention.

Bien sûr, l'invention n'est pas limitée aux exemples qui viennent d'être décrits et de nombreux aménagements peuvent être apportés à ces exemples sans sortir du cadre de l'invention.

## Revendications

1. Procédé de mesure et de caractérisation en ligne d'une biomasse dans un processus de fermentation de bactéries lactiques, en suspension dans un milieu, ce procédé comprenant :
- une mesure de la capacitance C dudit milieu pour une pluralité de fréquences,
- une mesure de la densité optique dudit milieu, pour ladite pluralité de fréquences, et
- un traitement en temps réel des signaux de mesure de capacitance et de densité optique par résolution d'un système dans lequel la capacitance C et la densité optique D sont chacune des fonctions de la concentration de biomasse X et de la taille des bactéries lactiques Φ pour déterminer simultanément la concentration de biomasse X et la taille des bactéries lactiques Φ.

## Patentansprüche

1. Verfahren zur On-line-Messung und -Charakterisierung einer Biomasse in einem Fermentationsprozess von Milchsäurebakterien in Suspension in einem Medium, wobei dieses Verfahren umfasst:
- eine Messung der Kapazität C des Mediums für eine Vielzahl von Frequenzen,
- eine Messung der optischen Dichte des Mediums für die Vielzahl von Frequenzen und
- eine Echtzeitverarbeitung der Signale der Messung von Kapazität und optischer Dichte durch Auflösung eines Systems, bei dem die Kapazität C und die optische Dichte D jeweils Funktionen der Biomasse-Konzentration X und der Größe der Milchsäurebakterien Φ sind, um die Biomasse-Konzentration X und die Größe der Milchsäurebakterien Φ gleichzeitig zu bestimmen.

## Claims

1. Method for the on-line characterization and measurement of a biomass in a lactic bacteria fermentation process, in suspension in a medium, this method comprising:
- measuring the capacitance C of said medium for a plurality of frequencies,
- measuring the optical density of said medium, for said plurality of frequencies, and
- processing in real time the capacitance and optical density measurement signals by resolution of a system in which the capacitance C and the optical density D are each functions of the biomass concentration X and of the size of the lactic bacteria Φ in order to determine simultaneously the biomass concentration X and the size of the lactic bacteria Φ.
